# EUROPEAN PATENT SPECIFICATION

(11) **EP 4 172 134 B1**
(45) Date of publication and mention of the grant of the patent: **12.08.2026**
(21) Application number: 21733836.7
(22) Date of filing: 23.06.2021
(51) Int. Cl.: C07C 29/80, C07C 31/04, B01D 3/14

(54) **PROCESS AND APPARATUS FOR DISTILLATION OF METHANOL**
VERFAHREN UND VORRICHTUNG ZUR DESTILLATION VON METHANOL
PROCÉDÉ ET APPAREIL DE DISTILLATION DE MÉTHANOL

(30) Priority: 29.06.2020 EP 20182805
(43) Date of publication of application: 03.05.2023
(73) Proprietor: Topsoe A/S, 2800 Kgs. Lyngby (DK)
(72) Inventor: SØRENSEN, Esben Lauge, 3400 Hillerød (DK); DAHL, Per Juul, 2950 Vedbæk (DK); CLARIDGE, Tais Bjerg, 25732 Rydebäck (SE); QUINTERO, Johannes, 2500 Valby (DK)
(74) Representative: Topsoe A/S
(86) International application number: PCT/EP2021/067158
(87) International publication number: WO 2022/002720

(56) References cited:
- WO-A1-2013/110369
- CN-A- 108 101 748

## Description

### Field of Application

The present invention is defined in the claims and refers to a process, apparatus and plant for distillation of methanol.

This method and apparatus may also be used to distillate other products such as ethanol.

### Background Art

It is known that the product of plants for synthesizing methanol, commonly defined as crude methanol, is an aqueous solution of methanol containing by-products of the synthesis reaction including ethanol, ketones, higher alcohols, and some dissolved gases including mainly H₂, CO, CO₂, N₂, CH₄.

The crude methanol is distilled to meet the purity specifications required on the market. For example, the grade AA specification requires a minimum methanol concentration of 99.85% by weight, wherein ethanol should not exceed 10 ppm by weight.

Known distillation processes are based substantially on one or more distillation columns, where typically at least one column is able to separate light products (for example gas) recovered at the top of the column from methanol, and at least one column is able to separate the heavier product (e.g. aqueous solution) recovered at the bottom of the column from methanol.

A specific process which is widely used for e.g. distillation of methanol, comprises two columns that operate at atmospheric pressure or close to atmospheric pressure. More specifically, said process uses a preliminary treatment column known as stabilizing column or pre-run column and a second distillation column. The first column substantially has the purpose of separating the more volatile components contained in the crude methanol, where it receives the crude methanol and separates the light components at the top and an aqueous solution at the bottom. The second column known as concentration column carries out the actual distillation, obtaining (i) refined methanol at the top, (ii) a prevalently aqueous stream at the bottom ("bottom water"), (iii) a side stream known as "fusel oil" mainly containing water, residual methanol (ca. 1 % of the total) and most of the by-products of the synthesis reaction. Said fusel oil has a certain heat value and is usually used as a fuel or feed in for synthesis gas generation.

Each column comprises a reboiler that heats the bottom of the column and maintains heat input to the distillation process. Each column comprises also a condenser, which condenses the top product and recycles it (at least partially) to said column. The heat is provided to the concentration (or distillation) column by steam, or by a process gas - when available - of suitable thermal level. The cooling medium for the condenser is normally water or air. Said configuration with two columns is simple in terms of a plant (e.g., a methanol distillation plant), but it has the major drawback of consuming a substantial amount of energy, both due to the heat supplied to the bottom reboilers, and due to the consumption of cooling water and/or electricity of the top condensers. Moreover, the columns have a relatively large diameter in relation to the production capacity and the plant cost is consequently high.

The order of magnitude of the heat consumption for the two bottom reboilers of about 3.35 x 10⁹ J (0.8 Gcal) per ton of refined methanol. Since the energy consumption necessary to produce a ton of crude methanol is about 25.10 x 10⁹ to 33.47 x 10⁹ J (6-8 Gcal), the order of magnitude of the energy consumption of the distillation is estimated to be 10% of the total consumption of the plant. The heat to be disposed of in the condensers is comparable with the heat exchanged in the reboilers. In the theoretical case, for example, of removing said heat exclusively with cooling water, the flow rate circulating is relevant, i.e. about 80 m3 per ton of methanol, and consequently there are high costs for pumping, etc.

There are other known distillation plants and processes that attempt to at least partially reduce these drawbacks.

US 4 210 495 describes a process with three distillation columns, i.e. a preliminary treatment or stabilizing column and two concentration columns, a column operating at a medium pressure of about 7-8 bar and a final concentration column, respectively. The stabilizing and final concentration columns operate substantially at atmospheric pressure or slightly higher pressure (e.g. 1 .5 bar). Such a configuration makes it possible to condense the top vapors of the medium pressure column in the bottom reboiler of the final column at atmospheric pressure, recovering heat, and this concept is known as "staggered columns" or "cascaded columns". The staggered column concept may be used several times, for example by letting a high pressure concentration column deliver heat to a concentration column operating at intermediate pressure, which in turn delivers heat to a column operating at low pressure. However, both the stabilizing column and the concentration column operating at highest pressure must be heated and consequently the specific consumption, whilst being lower than a plant with just two columns, is still high.

A further development of this idea was disclosed in WO2013110369, which describes a process in which the final distillation column is operating at the highest pressure thereby enabling the condensing of top vapors from the final concentration column to deliver heat to the reboiler for the stabilizing column. This configuration makes it possible to reduce the energy compared to the process configuration described in US 4 210 495, but this configuration has the significant drawback that all concentration columns will be operating at higher pressure than otherwise necessary and a further drawback of this configuration is that there is a mismatch between the stabilizer condenser duty and the final concentration column reboiler duty as the amount of heat required for the stabilizing column is much (~30-70%) lower than the amount of heat required for the final concentration column.

The implication of the mismatch between the stabilizer condenser duty and final concentration column reboiler duty is that either will part of the condensing duty for the final concentration column by supplied by a separate condenser cooled by air or water (extra cost and energy consumption) or the stabilizing column will become unnecessarily large (in diameter).

The drawback of the increased pressure in the concentration columns is two-fold. In the first place, increasing the concentration column pressures puts more severe specifications on the heat source so that a heat source, which is acceptable for heating up two staggered concentration columns may not be acceptable to heat up the three staggered columns that results from this configuration. A further drawback is that the separation efficiency in the concentration columns is reduced with increasing pressure. This in turn translates into an increased energy consumption for a given distillation column, as shown in Tables 1 and 2.

**Table 1 - Column feedstock, design and results achieved with the method and apparatus of the present invention.**

| **Column feed, mole %** | |
|---|---|
| MeOH | 66,61 |
| H2O | 33,00 |
| EtOH | 0,25 |
| Higher alcohols | 0,14 |
| **Number of equillibrium stages** | 49 |
| **Pressure drop across column, bar** | 0,9 |
| **MeOH recovery** | 98% |
| **MeOH purity** | 99,999% |
| **Water recovery** | 93% |
| **Water purity** | 99,998% |

**Table 2 - Required reboiler duty (J/h and Gcal/h) at different pressure to**

| Pressure, barg | Reboiler duty, J/h (Gcal/h) |
|---|---|
| 0,1 | 260.66 x 10⁹ (62,3) |
| 0,6 | 304,39 x 10⁹ (72,75) |
| 1,1 | 349,78 x 10⁹ (83,6) |
| 2,1 | 453,13 x 10⁹ (108,3) |

| | |
|---|---|
| Assuming 1 Gcal = 4.184 x 10⁹ Joules (J) | |

Document WO2013110369 addresses the problem of consuming a substantial amount of energy, both due to the heat supplied to the bottom reboilers, and due to the consumption of cooling water and/or electricity of the top condensers. It does not, however provide (or suggest) a solution to efficiently solve this problem when having more than 2 concentration columns for distillation of methanol, allowing for comparable savings of energy.

This document suggests that the adoption of a substantially higher bottoming pressure (i.e., at the final distillation column which would correspond to column V3 of the present invention) allows an energy saving and makes it possible to optimize the heat flows and that by increasing the bottoming pressure (which would correspond to P3 in the present invention), the distilled methanol in gaseous state, produced in the bottoming stage, has a substantially higher temperature than the temperature in the topping stage (which would correspond to stabilizing column V0 of the present invention) and sufficient to ensure that a stream of said distilled methanol can be used as a heat source for the preliminary topping step. This document claims to make it possible to reduce or eliminate the consumption of heat (for example from condensing steam) for the heating of the topping stage. It discloses that pressure p4, in the bottoming stage, is substantially higher than the topping pressure p1, so that the temperature of gaseous methanol, distilled in column 400, is substantially greater than the temperature of the liquid in the bottom of column 100. It also discloses that such temperature difference is at least 10 °C, i.e., the temperature of the gaseous methanol at the top of column 400 is at least 10 degrees Celsius higher than the temperature of the liquid in the bottom of column 100. This is what allows that at least part of said gaseous methanol can be used to heat, at least partially, the topping column 100.

CN108101748, and other documents, also mention an energy-saving process method and device for methanol purification, which is an energy-saving process and device for producing methanol from crude methanol using four towers and refers to a reduction of the operating energy consumption, however it is not clear or suggested how this is achieved and what are the process conditions.

### Description of the Invention

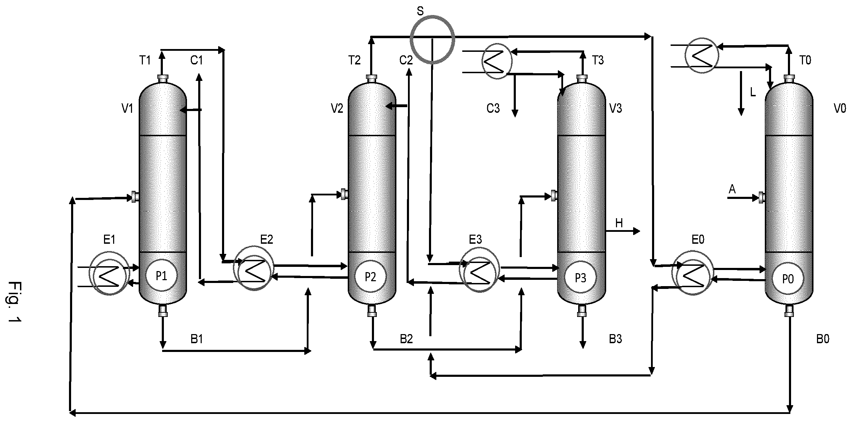

The present invention refers to an apparatus and process for distillation of methanol (Fig. 1), however may also be used in distillation of other products such as ethanol.

The present invention has the purpose of reducing the consumption of energy and of cooling water and/or electricity in a distillation process of crude intermediate products (e.g., methanol) comprising a pre-treatment stage, known as stabilizing stage, for the removal of the volatile components, and a concentration stage, comprising one or more columns for distillation.

Such a purpose is accomplished with a process for refining or distillation of a stream of crude methanol (A) comprising, in a preferred embodiment of the present invention:
(i) pre-treatment of a crude stream A of methanol in a stabilizing column V0 at pressure P0, for separation of volatile components, obtaining a stream of light gases L from the upper section of V0 and a liquid stream B0 comprising methanol from the lower section of V0,
(ii) B0 is then directed towards concentration column V1, at pressure P1,
(iii) gaseous stream T1 recovered from the upper section of V1 is condensed in heat exchanger E2, supplying energy to concentration column V2,
(iv) part of the condensed methanol obtained in step (iii) is sent to product C1, and the remaining part of the condensed methanol is added to the upper section of V1 and used as reflux flow,
(v) a liquid stream B1 comprising methanol is recovered from the lower section of V1 and passed on to V2, at pressure P2,
(vi) gaseous stream T2 recovered from the upper section of V2 is split (S) in two separate streams, one stream being condensed in a heat exchanger E0, supplying energy to V0, and the other stream being condensed in a heat exchanger E3, supplying energy to concentration column V3,
(vii) part of the condensed methanol obtained in step (vi) is sent to product, C2, and the remaining part of the condensed methanol is added to the upper section of V2 and used as reflux flow,
(viii) a liquid stream B2 comprising methanol is recovered from V2 and passed on to V3, at pressure P3,
(ix) gaseous stream T3, recovered from the upper section of V3, is condensed, part of the condensed methanol being sent to product, C3, and the remaining part being added to the upper section of V3 and used as reflux flow,
(x) one or more side streams H comprising higher alcohols and other minor bi-products is withdrawn from V3 and a liquid stream B3, is drawn from V3,
wherein:
- Columns V1, V2 and V3 operate at decreasing pressures, such that P1>P2>P3
- P0> 0 barg and P1> 0 barg
- each column V0, V1, V2 and V3 is correspondingly associated to a heat exchanger E0, E1, E2 and E3, which is a reboiler for the same column,
- heat exchangers E0 and E3 are condensers for column V2;
- heat exchanger E2 is condenser for column V1.
characterized in that:
- P1<2 barg; and
- Heat exchanger E1 is supplied by an external energy source.

And also an apparatus for distillation of methanol, comprising a stabilizing column V0, at pressure P0, connected in series with at least 3 distillation columns V1, V2 and V3 at correspondingly decreasing pressure P1, P2 and P3 wherein each column is associated to a heat exchanger E0, E1, E2 and E3, said heat exchanger being a reboiler for that column, characterized in that,
a) heat exchangers E0 and E3 are condensers of column V2;
b) heat exchanger E2 is condenser of column V1;.
c) E1 has an incoming heat stream, external to said apparatus;
d) P1< 2 barg,
as well as a plant for distillation of methanol, comprising at least one apparatus according to the present invention, wherein the amount of steam required for E1 is less than 1.3 kg/kg of product methanol and also the use of said apparatus for distillation of methanol.

The process of the present invention preferably comprises at least 4 columns, V0, V1, V2 and V3, but may comprise more concentration columns, from V1 up to Vn, operating at decreasing pressure, preferably connected in series.

Said concentration columns all operate in a manner such that part of the distillated product, C1, C2, C3 and so forward, is recovered from the upper section of each of the concentration columns, and the remaining liquid, B0, B1, B2, B3 and so forward, is passed on to the other column(s) until the final concentration column, e.g. in the preferred embodiment of the present invention said column is V3, from which the final fraction of the product C3 is recovered from the upper section, stream B3 comprising mainly water is recovered from the bottom and a mixture frequently referred to as fusel oil is recovered from a side stream H from the last concentration column - typically taken out in between the feed tray and the bottom of the column.

The heat exchange preferably takes place in a condenser/reboiler E0, E1, E2 and E3. Said heat exchanger may be a tube bundle or plate exchanger, in which the distilled methanol condenses in the hot side, and the solution evaporates in the cold side.

Preferably, the pressure of the condensers for the stabilizing step and low pressure concentration column are both slightly above atmospheric pressure, for example 0.1 - 0.5 bar thereby typically leading to a pressure in the reboiler for the stabilizing step of 0.5-1.5 bar, and the pressure in the condenser for the concentration column which delivers heat is at least 2 bar. Consequently, the pressure in the reboiler for the column which delivers heat is in the range 2 - 8 bar; more preferably about 7 bar.

Some embodiments comprise more than one concentration columns and several pressure levels but the column with the second lowest pressure level always supplies the heat to the stabilizing step.

The described pre-treatment (stabilizing) and concentration stages can be implemented using a single column or many columns in parallel, if necessary.

The final concentration column V3 produces distilled methanol C3 , a solution B3 mainly made up of water, and can also produce a side stream H represented by the so-called fusel oil. Side streams of fusel oil can also be extracted, if suitable, from the intermediate distillation stages.

The relative volatility between methanol and ethanol is highest at low pressure, so it is an advantage to conduct the separation at a pressure as low as possible.

Each time a column 1 receives heat from another column 2, the temperature level in column 1 increases and therefore also the column 2 pressure needs to be higher than in column 1. This is necessary to obtain the desirable driving force in the heat exchangers which act as condenser for the warmer column and as reboiler for the colder column. This temperature difference should be preferably be 4-10 °C. The temperature level of the external heat source (typically steam) moreover has preferably to be 4-10 °C higher than the temperature level in the bottom of the column with highest temperature level. Some prior art documents show staggering of all the concentration columns on top of the stabilizing column, they will end up with a higher demand for the temperature level of the external heat source than in the present invention, where the final concentration column is working at the same pressure level and therefore also essentially the same temperature level as the stabilizing column.

### Brief Description of Drawings

Figure 1 represents a preferred embodiment of the process for distillation of methanol according to the present invention, where three concentration columns V1, V2 and V3 with decreasing pressures P1>P2>P3 are connected to a stabilizing column V0 at pressure P0, wherein:
a. A: Feed of crude methanol
b. V0: Stabilizing column at pressure P0
c. V1, V2, V3: Concentration columns at pressure P1>P2>P3
d. B0: Liquid stream comprising methanol, recovered from the lower part of stabilizing column V0,
e. B1, B2, B3: Liquid streams comprising methanol, recovered from the lower part of concentrations columns V1, V2, V3,
f. C1, C2, C3: Product liquid distilled methanol,
g. E0, E1, E2, E3: Heat exchangers,
h. T0: Gaseous stream recovered from the upper part of stabilizing column V0
i. T1, T2, T3: Gaseous streams recovered from the upper part of concentration columns V1, V2, V3
j. L: Light gaseous stream from V0,
k. H: Side stream recovered from V3,
l. S: Split of streams.

### Definitions

"Atmospheric pressure" means 1,01325 bar, i.e., approximately 1 bar.

"Concentration column" or "distillation column" or "bottoming column", Vn where n≠0 (such as V1, V2 or V3), means a column divided into a series of stages. These correspond to a cascade of equilibrium stages. Liquid flows down the column from stage to stage and is contacted by vapor flowing upward. Traditionally, most columns have been built from a set of distinct "trays" or "plates", so these terms end up being essentially interchangeable with "stages". Each tray in a distillation column is designed to promote contact between the vapor and liquid on the stage. Distillation can be conducted in a packed column (just as absorption can be done in a trayed column). Stages may be numbered from top down or bottom up. The stream recovered from the upper section of the column is called the overhead product, T, the "overhead", the "top product". Distillate product C may be liquid or vapor (or occasionally both) depending on the type of condenser used. The distillate flow rate is herein designated C and the product recovered from the lower section of the column may be called the bottom product and is herein given the symbol B. In some situations, one or more "sidedraw" products H may be removed from the column. The portion of the column above the feed tray is called the rectification section. In this section, the vapor is enriched by contact with the reflux. The portion of the column below the feed tray is called the stripping section. The liquid portion of the feed serves as the reflux for this section. The operating pressure of the column, Pn (such as P1, P2 and P3) is typically controlled by adjusting heat removal in the heat exchanger. The base of the column is typically used as a reservoir to hold liquid leaving the bottom tray. A heat exchanger, e.g. a reboiler, is used to boil this liquid. The vapor which results, the "boilup", is returned to the bottom of the column.

A "Condenser" is required to provide the cooling duty to condense the stream collected from the upper section of the concentration column, which is a gas or vapor. The condensed vapor is partially refluxed back into the top of the column to increase the sharpness of the separation - the greater the reflux ratio the better is the separation. The condenser can be a total condenser or a partial condenser. In a total condenser, all the top vapor product is condensed to liquid, whereas in a partial condenser, only part of the vapor is condensed, and the liquid is refluxed back to the column, the un-condensed vapor is drawn for further processing. In this case, the partial condenser can be viewed as an additional VLE separation stage, whereas in a total condenser the top product of the column has the same composition as the reflux stream.

"Crude methanol", A, means a solution comprising methanol, typically 65 to 95% methanol, water and other components. The crude methanol A contains low-boiling and high-boiling components (light and heavy ends). The light ends L include mainly dissolved gases (e.g., CO2), dimethyl ether, methyl formate, and acetone. The heavy ends H include higher alcohols, long-chain hydrocarbons, higher ketones, and esters of lower alcohols with formic, acetic, and propionic acids.

"Distillation" or "fractional distillation" or "fractionation" means a process for separating liquid mixtures into two or more vapor or liquid products with different compositions. Distillation is an equilibrium stage operation. In each stage, a vapor phase is contacted with a liquid phase and mass is from vapor to liquid and from liquid to vapor. The less volatile, "heavy" or "high boiling", components concentrate in the liquid phase; the more volatile, "light", components concentrate in the vapor. By using multiple stages in series with recycle, separation can be accomplished. The feed to a distillation column may be liquid, vapor, or a liquid-vapor mixture. It may enter at any point in the column. More than one stream may be fed to the system, and more than one product may be drawn. Vapor leaving the top of the column, such as T0, T1, T2 and T3, passes through a heat exchanger, where it is partially or totally condensed. The resulting liquid is temporarily held in the "accumulator" or reflux drum. A liquid stream is withdrawn from the drum and returned to the top tray of the column as reflux to promote separation.

"Equilibrium stage" or theoretical plate means, in many separation processes such as distillation, a hypothetical zone or stage in which two phases, such as the liquid and vapor phases of a substance, establish an equilibrium with each other. Such equilibrium stages may also be referred to as an ideal stage or a theoretical tray. The performance of many separation processes depends on having series of equilibrium stages and is enhanced by providing more such stages. In other words, having more equilibrium stages increases the efficiency of the separation process.

"Feed" or "column feed" to a distillation column may be liquid, vapor, or a liquid-vapor mixture. It may enter at any point in the column, although the optimal feed tray location should be determined and used. More than one stream may be fed to the system, and more than one product may be drawn. The thermal condition of the feed determines the column internal flows. If the feed is below its boiling point, heat is needed to raise it to where it can be vaporized. This heat must be obtained by condensing vapor rising through the column, so the liquid flow moving down the column increases by the entire amount of the feed plus the condensed material and the vapor flow upward is decreased.

"Gaseous stream, such as T0, T1, T2 or T3, recovered from the upper part of stabilizing column V0 and concentration columns, such as V1, V2 or V3, is the stream resulting from a distillation process, taken from the upper part of said columns. Such a stream is mainly made up of methanol, with progressively lower content of impurities until the gaseous stream, such as T3, recovered from the last concentration column, such as V3, has a very low content of impurities. The requirement is that the mixed product streams, C1 + C2 +C3, shall fulfil the required product specification (e.g. grade AA).

"Heat duty" or "Duty" means the amount of heat needed to transfer from a hot side to the cold side over a unit of time. The equation to calculate the heat duty is normally written in two ways: a) one that can be used for sensible heat transferred, which means that the fluid undergoes no phase change; b) the other can be used for latent heat transferred, which means that the fluid undergoes a phase change. i.e. condenses.

"Heat exchanger" means a system used to transfer heat between two or more fluids. Heat exchangers are used in both cooling and heating processes. The fluids may be separated by a solid wall to prevent mixing or they may be in direct contact. In particular, "heat exchanger" means a reboiler/condenser, e.g. E0, E1, E2 and E3, such as a tube bundle exchanger, for example with evaporation of the solution in the shell side and condensation of the distillate in the tube side (or vice-versa). It is also possible to use a plated heat exchanger with heat exchange plates housed inside a shell.

"Partial reboiler" means a reboiler where only part of the liquid in the column base is vaporized. The vapor produced is returned to the column, and the liquid stream is removed as product or feed to an additional column . The compositions of these three streams are different. Partial reboilers also provide an ideal separation stage. Sidestream reboilers can be used, which draw liquid off a tray, heat it, and then return the vapor liquid mixture to the same or similar trays.

"Heavy by-products" or "side streams", H, means a stream comprising higher alcohols and other minor bi-products recovered from the last concentration column - typically taken out in between the feed tray and the bottom of the column. It is also known as "fusel oil" and comprises water, residual methanol (ca. 1 % of the total) and most of the by-products of the synthesis reaction. Said fusel oil has a certain heat value and is usually used as a fuel or feed to a synthesis gas generation section. Side streams of fusel oil can also be extracted, if suitable, from the intermediate distillation stages.

"Intermediate products" mean vapor and liquid streams between columns and exiting columns in a multi column distillation apart from the feed stream A, the product streams C1, C2, C3, L, H and B3.

"Light by-products" or "Light gases" or "Light ends", L, mean gaseous stream obtained from stabilizing column V0, preferably the upper section of V0 and includes mainly dissolved gases (e.g., CO2), dimethyl ether, methyl formate, and acetone.

"Liquid stream comprising methanol", such as B0, B1, B2 or B3 means a stream recovered from the lower section of stabilizing column V0 and concentration columns, such as V1, V2 and V3. As the distillation progresses, this stream gradually comprises a lower amount of methanol and a higher amount of water, until the last stream, such as B3, recovered from the last concentration column, such as V3, comprises mainly water and only residual amounts of methanol, preferably less than 50 ppm.

"Lower stream" or "bottom stream" means a stream obtained or recovered from the lower section of a column, such as V0, V1, V2 and V3.

"Pressure", P, means gauge pressure and is measured in bar(g). Gauge pressure is the pressure relative to atmospheric pressure and it is positive for pressures above atmospheric pressure, and negative for pressures below it. The difference between bar and bar(g) is the difference in the reference considered. Measurement of pressure is always taken against a reference and corresponds to the value obtained in a pressure measuring instrument. If the reference in the pressure measurement is vacuum we obtain absolute pressure and measure it in bar only. If the reference is atmospheric pressure then pressure is cited in bar(g).

"Product", such as C1, C2 or C3, is liquid distilled methanol recovered from concentration columns such as V1, V2 and V3.

"Reboiler" means a heat exchanger typically used to provide heat to the bottom of industrial distillation columns. Reboilers boil the liquid from the bottom of a distillation column to generate vapors which are returned to the column, such as V0, V1, V2 and V3, to drive the distillation separation. The heat supplied to the column by the reboiler at the bottom of the column is removed by the condenser at the top of the column. Most reboilers are of the shell and tube heat exchanger type and normally steam is used as the heat source in such reboilers. However, other heat transfer fluids like hot synthesis gas, oil or Dowtherm (TM) may be used. Fuel-fired furnaces may also be used as reboilers in some cases.

"Split of streams", S, means separation of at least one original stream (liquid or gaseous) in two separate streams or sub-streams (liquid or gaseous). Within the context of the present invention, S indicates the location where gaseous stream T2 recovered from the upper section of V2 is divided in two deriving streams or sub-streams, one stream or sub-stream being condensed in a heat exchanger E0, supplying energy to V0, and the other stream or sub-stream being condensed in a heat exchanger E3, supplying energy to concentration column V3.

"Stabilizing column" or Topping column or pre-run column or, V₀, is for separating the more volatile components from the heavier components, both contained in the crude product, such as crude methanol.

"Volatile components" or "volatile substances" means components or substances which vaporize readily at low temperatures. Volatility can also describe the tendency of a vapor to condense into a liquid or solid: less volatile substances will more readily condense from a vapor than highly volatile ones. *Vapor pressure* is a measurement of how readily a condensed phase forms a vapor at a given temperature. A substance enclosed in a sealed vessel initially at vacuum (no air inside) will quickly fill any empty space with vapor. After the system reaches equilibrium and no more vapor is formed, this vapor pressure can be measured. Increasing the temperature increases the amount of vapor that is formed and thus the vapor pressure. In a mixture, each substance contributes to the overall vapor pressure of the mixture, with more volatile compounds making a larger contribution. *Boiling point* is the temperature at which the vapor pressure of a liquid is equal to the surrounding pressure, causing the liquid to rapidly evaporate, or boil. It is closely related to vapor pressure, but is dependent on pressure. The normal boiling point is the boiling point at atmospheric pressure, but it can also be reported at higher and lower pressures.

"Upper stream" or "Top stream" means a stream obtained or recovered from the upper section of a column, such as V0, V1, V2 and V3.

## Claims

1. Process for distillation of methanol, comprising:
(i) pre-treatment of a crude stream A of methanol in a stabilizing column V0 at pressure P0, for separation of volatile components, obtaining a stream of light gases L from the upper section of V0 and a liquid stream B0 comprising methanol from the lower section of V0,
(ii) B0 is then directed towards concentration column V1, at pressure P1,
(iii) gaseous stream T1 recovered from the upper section of V1 is condensed in heat exchanger E2, supplying energy to concentration column V2,
(iv) part of the condensed methanol obtained in step (iii) is sent to product C1, and the remaining part of the condensed methanol is added to the upper section of V1 and used as reflux flow,
(v) a liquid stream B1 comprising methanol is recovered from the lower section of V1 and passed on to V2, at pressure P2,
(vi) gaseous stream T2 recovered from the upper section of V2 is split (S) in two separate streams, one stream being condensed in a heat exchanger E0, supplying energy to V0, and the other stream being condensed in a heat exchanger E3, supplying energy to concentration column V3,
(vii) part of the condensed methanol obtained in step (vi) is sent to product, C2, and the remaining part of the condensed methanol is added to the upper section of V2 and used as reflux flow,
(viii) a liquid stream B2 comprising methanol is recovered from V2 and passed on to V3, at pressure P3,
(ix) gaseous stream T3, recovered from the upper section of V3, is condensed, part of the condensed methanol being sent to product, C3, and the remaining part being added to the upper section of V3 and used as reflux flow,
(x) one or more side streams H comprising higher alcohols and other minor biproducts is withdrawn from V3 and a liquid stream B3, is drawn from V3,
wherein:
- Columns V1, V2 and V3 operate at decreasing pressures, such that P1>P2>P3
- P0> 0 barg and P1> 0 barg
- each column V0, V1, V2 and V3 is correspondingly associated to a heat exchanger E0, E1, E2 and E3, which is a reboiler for the same column,
- heat exchangers E0 and E3 are condensers for column V2;
- heat exchanger E2 is condenser for column V1.
**characterized in that**:
- P1<2 barg; and
- Heat exchanger E1 is supplied by an external energy source.

2. Process according to claim 1, wherein the temperature in the coldest part of V2 is higher than the temperature in the warmest part of V3 and V0 and the temperature in the coldest part of V1 is higher than the temperature in the warmest part of V2.

3. Process according to claims 1 and 2 wherein the temperature in the coldest part of V2 is preferably 4 °C higher than the temperature in the warmest part of V3 and V0 and the temperature in the coldest part of V1 is preferably 4 °C higher than the temperature in the warmest part of V2.

4. Process according to the previous claims wherein the difference between P1 and P0 is greater than or equal to 7.7 bar.

5. Process according to the previous claims, wherein P1 is higher than 9,7 bar(g), P2 is comprised between 6,9 and 13 bar(g).

6. Process according to the previous claims wherein P1 is preferably 17 bar(g), P2 is preferably 9 bar(g) and P3 is preferably 0,98 bar(g).

7. Process according to the previous claims wherein the heat duty of E0 is at least 30% less than the duty of E1.

8. Process according to the previous claims wherein B3 comprises the water removed from circulating streams and is recovered from the lower section of V3.

9. Apparatus for distillation of methanol, comprising a stabilizing column V0, at pressure P0, connected in series with at least 3 distillation columns V1, V2 and V3 at correspondingly decreasing pressure P1, P2 and P3 wherein each column is associated to a heat exchanger E0, E1, E2 and E3, said heat exchanger being a reboiler for that column, **characterized in that**,
a) heat exchangers E0 and E3 are condensers of column V2;
b) heat exchanger E2 is condenser of column V1;
c) E1 has an incoming heat stream, external to said apparatus;
d) P1< 2 barg.

10. Apparatus according to claim 9 wherein the external heat stream to E1 is steam or a synthesis gas containing sensible heat.

11. Plant for distillation of methanol according to claims 1 to 8 comprising at least one apparatus according to claims 9-10, wherein the amount of steam required for E1 is less than 1.3 kg/kg of product methanol.

12. Use of apparatus according to claims 9-10 in a plant according to claim 11, for distillation of methanol according to claims 1-8.

## Patentansprüche

1. Verfahren zur Destillation von Methanol, umfassend:
(i) Vorbehandlung eines Rohstroms A von Methanol in einer Stabilisierungssäule VO bei Druck PO, zur Abscheidung flüchtiger Komponenten, wodurch ein Strom leichter Gase L aus dem oberen Abschnitt von V0 und ein flüssiger Strom B0, der Methanol umfasst, aus dem unteren Abschnitt von V0 erhalten werden,
(ii) B0 wird dann bei Druck P1 zur Konzentrationssäule V1 geleitet,
(iii) der aus dem oberen Abschnitt von V1 gewonnene Gasstrom T1 wird im Wärmetauscher E2 kondensiert, wodurch Konzentrationssäule V2 Energie zugeführt wird,
(iv) ein Teil des in Schritt (iii) erhaltenen kondensierten Methanols wird zum Produkt C1 geschickt, und der verbleibende Teil des kondensierten Methanols wird dem oberen Abschnitt von V1 zugesetzt und als Rückflussstrom verwendet,
(v) ein flüssiger Strom B1, der Methanol umfasst, wird aus dem unteren Abschnitt von V1 gewonnen und bei Druck P2 an V2 weitergeleitet,
(vi) der aus dem oberen Abschnitt von V2 gewonnene gasförmige Strom T2 wird in zwei getrennte Ströme aufgeteilt (S), wobei ein Strom in einem Wärmetauscher E0 kondensiert wird, wodurch V0 Energie zugeführt wird, und der andere Strom in einem Wärmetauscher E3 kondensiert wird, wodurch der Konzentrationssäule V3 Energie zugeführt wird,
(vii) ein Teil des in Schritt (vi) erhaltenen kondensierten Methanols wird zum Produkt C2 geschickt, und der verbleibende Teil des kondensierten Methanols wird dem oberen Abschnitt von V2 zugesetzt und als Rückflussstrom verwendet,
(viii) ein flüssiger Strom B2, der Methanol umfasst, wird aus V2 gewonnen und bei Druck P3 an V3 weitergeleitet,
(ix) der gasförmige Strom T3, der aus dem oberen Abschnitt von V3 gewonnen wird, wird kondensiert, wobei ein Teil des kondensierten Methanols zum Produkt C3 geschickt wird und der verbleibende Teil dem oberen Abschnitt von V3 zugesetzt und als Rückflussstrom verwendet wird,
(x) ein oder mehrere Seitenströme H, die höhere Alkohole und andere kleinere Nebenprodukte umfassen, werden aus V3 abgezogen und ein flüssiger Strom B3 wird aus V3 abgezogen,
wobei:
- Säulen V1, V2 und V3 bei abnehmenden Drücken arbeiten, so dass P1>P2>P3 P0>0 barg und P1>0 barg
- jede Säule V0, V1, V2 und V3 entsprechend einem Wärmetauscher E0, E1, E2 und E3 zugeordnet wird, der ein Verdampfer für die gleiche Säule ist,
- Wärmetauscher E0 und E3 Kondensatoren für Säule V2 sind;
- Wärmetauscher E2 Kondensator für Säule V1 ist.
**dadurch gekennzeichnet, dass**:
- P1<2 barg; und
- Wärmetauscher E1 von einer externen Energiequelle versorgt wird.

2. Verfahren nach Anspruch 1, wobei die Temperatur im kältesten Teil von V2 höher ist als die Temperatur im wärmsten Teil von V3 und V0, und die Temperatur im kältesten Teil von V1 höher ist als die Temperatur im wärmsten Teil von V2.

3. Verfahren nach den Ansprüchen 1 und 2, wobei die Temperatur im kältesten Teil von V2 vorzugsweise 4 °C höher ist als die Temperatur im wärmsten Teil von V3 und V0, und die Temperatur im kältesten Teil von V1 vorzugsweise 4 °C höher ist als die Temperatur im wärmsten Teil von V2.

4. Verfahren nach den vorstehenden Ansprüchen, wobei die Differenz zwischen P1 und P0 größer oder gleich 7,7 bar ist.

5. Verfahren nach den vorstehenden Ansprüchen, wobei P1 höher als 9,7 bar(g) ist, wobei P2 zwischen 6,9 und 13 bar(g) liegt.

6. Verfahren nach den vorstehenden Ansprüchen, wobei P1 vorzugsweise 17 bar(g) ist, P2 vorzugsweise 9 bar(g) ist und P3 vorzugsweise 0,98 bar(g) ist.

7. Verfahren nach den vorstehenden Ansprüchen, wobei die Wärmeleistung von E0 mindestens 30 % niedriger ist als die Leistung von E1.

8. Verfahren nach den vorstehenden Ansprüchen, wobei B3 das aus zirkulierenden Strömen entfernte Wasser umfasst und aus dem unteren Abschnitt von V3 gewonnen wird.

9. Einrichtung zur Destillation von Methanol, umfassend eine Stabilisierungssäule V0 bei Druck P0, die mit mindestens 3 Destillationssäulen V1, V2 und V3 bei entsprechend abnehmendem Druck P1, P2 und P3 in Reihe verbunden ist, wobei jede Säule einem Wärmetauscher E0, E1, E2 und E3 zugeordnet ist, wobei der Wärmetauscher ein Verdampfer für diese Säule ist, **dadurch gekennzeichnet, dass**
a) Wärmetauscher E0 und E3 Kondensatoren der Säule V2 sind;
b) Wärmetauscher E2 Kondensator der Säule V1 ist;
c) E1 einen eingehenden Wärmestrom außerhalb der Einrichtung aufweist;
d) P1<2 barg.

10. Einrichtung nach Anspruch 9, wobei der äußere Wärmestrom zu E1 Dampf oder ein Synthesegas ist, das fühlbare Wärme enthält.

11. Anlage zur Destillation von Methanol nach den Ansprüchen 1 bis 8, umfassend mindestens eine Einrichtung nach den Ansprüchen 9-10, wobei die für E1 erforderliche Dampfmenge weniger als 1,3 kg/kg an Produktmethanol beträgt.

12. Verwendung einer Einrichtung nach den Ansprüchen 9-10 in einer Anlage nach Anspruch 11 zur Destillation von Methanol nach den Ansprüchen 1-8.

## Revendications

1. Procédé de distillation de méthanol, comprenant :
(i) le prétraitement d'un courant brut A de méthanol dans une colonne de stabilisation V0 à une pression P0, pour séparation de composants volatils, obtenant un courant de gaz légers L provenant de la partie supérieure de V0 et un courant liquide B0 comprenant du méthanol provenant de la partie inférieure de V0,
(ii) B0 est ensuite dirigé vers une colonne de concentration V1, à une pression P1,
(iii) un courant gazeux T1 récupéré de la section supérieure de V1 est condensé dans un échangeur de chaleur E2, effectuant l'alimentation en énergie à une colonne de concentration V2,
(iv) une partie du méthanol condensé obtenu à l'étape (iii) est envoyée à un produit C1, et la partie restante du méthanol condensé est ajoutée à la section supérieure de V1 et utilisée en tant qu'écoulement de reflux,
(v) un courant liquide B1 comprenant du méthanol est récupéré de la section inférieure de V1 et transféré à V2, à une pression P2,
(vi) un courant gazeux T2 récupéré de la section supérieure de V2 est divisé (S) en deux courants séparés, un courant étant condensé dans un échangeur de chaleur E0, effectuant l'alimentation en énergie à V0, et l'autre courant étant condensé dans un échangeur de chaleur E3, effectuant l'alimentation en énergie à une colonne de concentration V3,
(vii) une partie du méthanol condensé obtenu à l'étape (vi) est envoyée à un produit, C2, et la partie restante du méthanol condensé est ajoutée à la section supérieure de V2 et utilisée en tant qu'écoulement de reflux,
(viii) un courant liquide B2 comprenant du méthanol est récupéré de V2 et transféré à V3, à une pression P3,
(ix) un courant gazeux T3, récupéré de la section supérieure de V3, est condensé, une partie du méthanol condensé étant envoyée à un produit, C3, et la partie restante étant ajoutée à la section supérieure de V3 et utilisée en tant qu'écoulement de reflux,
(x) un ou plusieurs courants latéraux H comprenant des alcools de haut poids moléculaire et d'autres sous-produits mineurs sont soutirés de V3 et un courant liquide B3 est soutiré de V3,
dans lequel :
- les colonnes V1, V2, et V3 fonctionnent à des pressions décroissantes, telles que P1 > P2 > P3,
P0 > 0 barg et P1 > 0 barg,
- chaque colonne V0, V1, V2, et V3 est associée de façon correspondante à un échangeur de chaleur E0, E1, E2, et E3, qui est un rebouilleur pour la même colonne,
- des échangeurs de chaleur E0 et E3 sont des condenseurs pour la colonne V2 ;
- l'échangeur de chaleur E2 est un condenseur pour la colonne V1 ;
**caractérisé en ce que** :
- P1 < 2 barg ; et
- l'échangeur de chaleur E1 est alimenté par une source d'énergie externe.

2. Procédé selon la revendication 1, dans lequel la température dans la partie la plus froide de V2 est supérieure à la température dans la partie la plus chaude de V3 et de V0, et la température dans la partie la plus froide de V1 est supérieure à la température dans la partie la plus chaude de V2.

3. Procédé selon les revendications 1 et 2, dans lequel la température dans la partie la plus froide de V2 est de préférence supérieure de 4 °C à la température dans la partie la plus chaude de V3 et de V0, et la température dans la partie la plus froide de V1 est de préférence supérieure de 4 °C à la température dans la partie la plus chaude de V2.

4. Procédé selon les revendications précédentes, dans lequel la différence entre P1 et P0 est supérieure ou égale à 7,7 bar.

5. Procédé selon les revendications précédentes, dans lequel P1 est supérieure à 9,7 bar(g), P2 est comprise entre 6,9 et 13 bar(g).

6. Procédé selon les revendications précédentes, dans lequel P1 est de préférence 17 bar(g), P2 est de préférence 9 bar(g) et P3 est de préférence 0,98 bar(g).

7. Procédé selon les revendications précédentes, dans lequel la demande calorifique de E0 est au moins 30 % inférieure à la demande de E1.

8. Procédé selon les revendications précédentes, dans lequel B3 comprend l'eau retirée de courants de circulation et est récupérée de la section inférieure de V3.

9. Appareil de distillation de méthanol, comprenant une colonne de stabilisation V0, à une pression P0, raccordée en série à au moins 3 colonnes de distillation V1, V2, et V3 à une pression décroissante de façon correspondante P1, P2, et P3, dans lequel chaque colonne est associée à un échangeur de chaleur E0, E1, E2, et E3, ledit échangeur de chaleur étant un rebouilleur pour cette colonne, **caractérisé en ce que**,
a) les échangeurs de chaleur E0 et E3 sont des condenseurs de la colonne V2 ;
b) l'échangeur de chaleur E2 est un condenseur de la colonne V1 ;
c) E1 présente un courant de chaleur entrant, externe audit appareil ;
d) P1< 2 barg.

10. Appareil selon la revendication 9, dans lequel le courant de chaleur externe pour E1 est de la vapeur ou un gaz de synthèse contenant de la chaleur sensible.

11. Installation, pour distillation de méthanol selon les revendications 1 à 8, comprenant au moins un appareil selon les revendications 9-10, dans laquelle la quantité de vapeur requise pour E1 est inférieure à 1,3 kg/kg du méthanol-produit.

12. Utilisation d'un appareil selon les revendications 9-10 dans une installation selon la revendication 11, pour distillation de méthanol selon les revendications 1-8.
